# EUROPEAN PATENT SPECIFICATION

(11) **EP 0 394 734 B1**
(45) Date of publication and mention of the grant of the patent: **15.02.1995**
(21) Application number: 90106784.3
(22) Date of filing: 09.04.1990
(51) Int. Cl.: A61L 2/20, B65B 55/10

(54) **A method and an apparatus for sterilizing objects by means of a gaseous sterilization agent**
Verfahren und Vorrichtung zur Sterilisation mit Hilfe von Sterilisiergas
Procédé et appareil de stérilisation au moyen d'un agent gazeux

(30) Priority: 25.04.1989 SE 8901498
(43) Date of publication of application: 31.10.1990
(73) Proprietor: Tetra Laval Holdings & Finance SA, 1009 Pully (CH)
(72) Inventor: Nilsson, Kjell Gottfrid, SE-24500 Staffanstorp (SE)
(74) Representative: Müller, Hans-Jürgen, Dipl.-Ing.

(56) References cited:
- EP-A- 0 302 420
- EP-A- 0 310 900
- GB-A- 1 148 645
- US-A- 4 374 087
- US-A- 4 752 445

## Description

The present invention relates to a method for sterilization objects, primarilly packaging material or packages produced thereof, for sterilized liquid foods, by means of a gaseous sterilization in accordance with the opening part of claim 1, and an apparatus for carrying out this method.

A method of this type is described in EP-A-0 310 900. The object to be sterilized according to this known method is a web which is forwarded through a sterilizing chamber into which the sterilizing agent is introduced and from which gases are removed by means of a vacuum source. However, this known method and apparatus is not suited for relatively large objects unless high amounts of sterilizing gas will be lost.

Furthermore, a relatively similar device is known (US-A-4 374 087) which is used for sterilizing mattresses, laundry sacks and similar articles within a relatively large chamber which has to be completely filled with the sterilizing agent. It is necessary to circulate high amounts of sterilizing agents.

Furthermore, it is known (GB-B-1 148 645) to sterilize rectangular packages for e.g. sterile milk within a hood which is to be disposed onto a plate which serves as a support for the package.

These so-called single-use packages or cartons are produced with the aid of modern, high capacity packaging machines of the type which, either from a continuous web or from prefabricated blanks of a laminated packaging material, normally plastic-coated paper, both form, fill and seal the packages. From a web, a packaging machine produces packages in that the web is first reformed into a tube, in that both longitudinal edges of the web are united with one another in an overlapping seam joint. The tube is filled with its intended contents and is divided into individual pad shaped package units by repeated transverse sealing of the tube transversely of the longitudinal axis of the tube, whereafter the package units are separated from one another by incisions in the transverse sealing zones, and are given the desired final geometric configuration, normally parallelipipedic, by inward folding and permanent sealing of the double-walled triangular corner flaps of the package units against the outside of the package.

From flat-laid tubular blanks, another prior art machine type produces packages by feeding the blanks, while raising them to open tubular form, one-by-one from a magazine disposed adjacent the machine out onto a moving conveyor belt which conveys the raised blanks to the first processing station of the machine proper, or bottom forming station. The bottom forming station includes one or more intermittently rotatable mandrel wheels with radial mandrels on which the advanced blanks are transported through a number of forming and sealing stations in order to give the blanks suitable bottom sealing. From the mandrel wheel, the blanks are transported through a filling station and a top forming station in which the blanks are given a suitable liquid-tight top seal.

For reasons of distribution logistics, it is often advantageous to sterilize a liquid food in order to prolong the shelf life of the food and thereby improve the basic preconditions for the capability to distribute the food rationally while retaining qualities of freshness even if distribution were to be effected over long transport distances and/or otherwise be time-consuming. In order not to jeopardise the contemplated superior shelf life of the food by sterilization, and also the distribution logistics advantages intimately linked therewith, it is, of course, important to ensure that the sterilized food is prevented from coming into contact with and being re-infected by bacteria. According to prior art technology, such an aseptic handling of the sterilized food is catered for in that the packaging material or packages produced thereof and ready for filling are sterilized, and that the filling process itself is carried out in a sterile atmosphere.

For example, a packaging material web is sterilized in that the web is led through a bath containing a hydrogen peroxide solution which is thereby brought into contact with and acts on the packaging material. This prior art sterilization method functions satisfactorily in webs with planar, smooth surfaces, but it has always been difficult to achieve efficient sterilization of webs with surface irregularities, for example so-called pull-tabs which are removably sealed over ready furnished pouring openings and which often occur on this type of material web. The sterilization difficulties are at least partly because of the fact that the web is located in contact with the sterilization agent for far too short a time to be able to penetrate in and act in inaccessible web regions at such surface irregularities. Another problem which is referrable to the fact that the sterilization is carried out in the liquid phase - and which is particularly manifest in the sterilization of packaging materials including one or more fibre layers - is the difficulty in avoiding so-called edge assimilation of sterilization agent in the exposed fibre regions of the material, for example along the both longitudinal section edges of the packaging material web which readily absorb moisture.

It is known to use as a gaseous sterilization agent water/hydrogen peroxide vapour which is brought into contact with a heated packaging material web in a substantially closed sterilization chamber. A sterilization process of web-chaped packages can avoid the above-discussed problems, but one basic precondition for attaining a good sterilization effect along all web regions, including inaccessible spaces of the above-described type, is that a high flow rate is imparted to the gas in relation to the packaging material web in order to ensure contact which is efficient from the point of view of sterilization between the sterilization gas and the packaging material web. The prior art gas sterilization method is primarily suitable for planar material webs, while, in practice, it has proved to be more difficult to use for sterilization of intermittently advanced objects, eg. packages produced from prefabricated package blanks.

It is an object of the present invention to easily sterilize articles being substantially larger than thin webs without a substantial loss of sterilizing agents in a realtively simple way avoiding problems and drawbacks of the types mentioned above.

The invention according to the method is characterized in claim 1 and in accordance with the apparatus in claim 3. Subclaims concern preferred embodiments of the invention which are also described in the following specification and by means of the drawings.

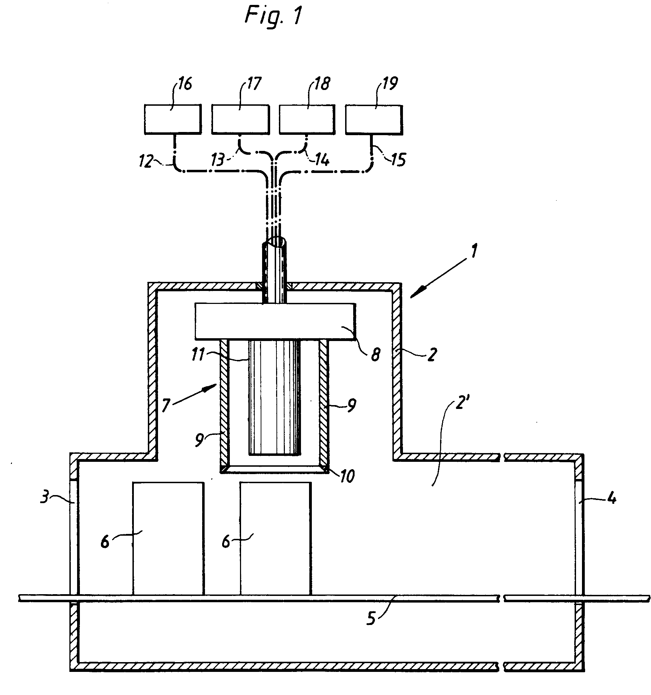

- Figures 1, 2 and 3: schematically illustrate an apparatus according to the present invention on different occasions during a sterilization cycle according to the invention.

As was intimated earlier, the method according to the present invention may be applied to practically any type whatever of object which is to be sterilized by means of a gaseous sterilization agent, but since the method has proved to be particularly usable in sterilization of the present invention will, solely for this reason, be described and illustrated with specific reference to such an advantageous physical application.

The apparatus illustrated in Figs. 1-3, which has been given the generic reference numeral 1, comprises a substantially completely closed outer chamber casing 2 with an inlet aperture 3 and an outlet aperture 4 for a conveyor belt 5 on which the objects like packages 6 which are to be sterilized are disposed to be conveyed through the chamber 2' defined by the casing 2. Within the chamber 2', there is provided a hood 7 which is disposed to be vertically displaceable above the conveyor belt 5 and comprises a hood top 8 and vertical hood walls 9 which are downwardly defined by a seal 10 disposed about the lower edge of the hood walls. Centrally within the hood 7, there is disposed one or more vertical pipes 11 discharging a short distance above the lower edge of the hood walls 9 and being, by outer connections 12-15, ideally connected with an outer vacuum source 16, a source 17 for sterilization gas, a recycling tank 18 for sterilization gas, and a source 19 for sterile air, respectively.

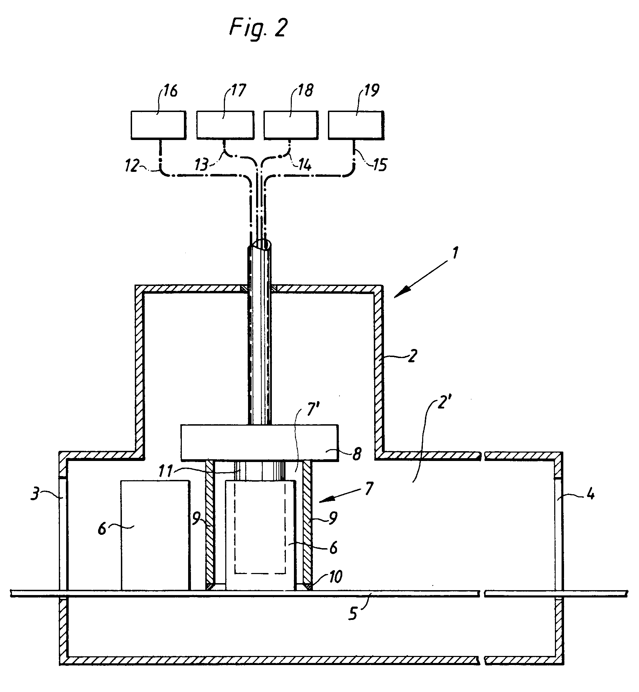

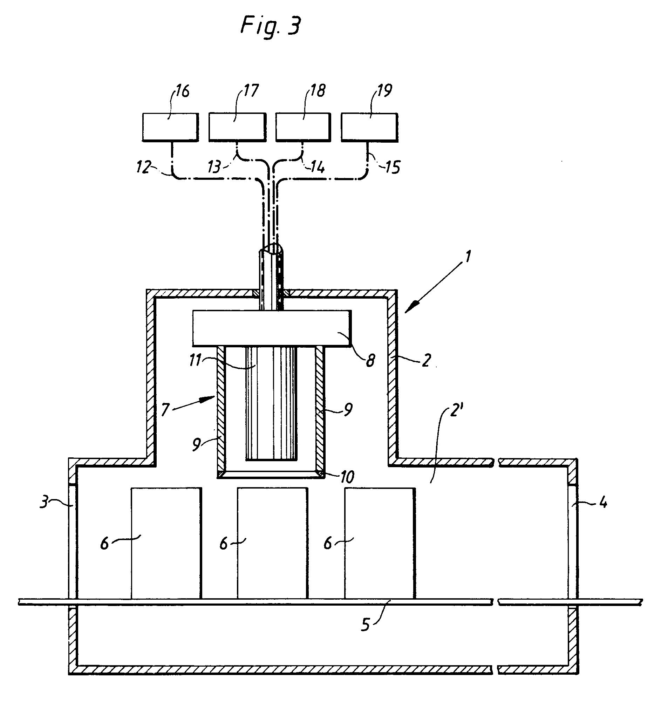

The apparatus 1 operates as follows. With the aid of the conveyor belt 5, the packages 6 are transported through the inlet aperture 3 into the outer chamber 2' to that position which is illustrated more closely in Fig. 1. The conveyor belt 5 stops momentarily and the sterilization hood 7 ideally disposed straight above the package 6 is lowered so that the seal 10 disposed about the lower edge of the hood walls 9 is sealingly abutted against the conveyor belt 5 serving now as the bottom of the hood 7, the package 6 being wholly enclosed in the sterilization chamber 7' formed by the hood top 8, the hood walls 9 and the conveyor belt 5, as illustrated in Fig. 2. The pipe 11 discharging centrally in the package 6 immediately above the bottom of the package is placed in communication with the outer vacuum source 12 through the connection 16 for evacuation and elimination of the boundary layer of air along the surfaces of the package 6 intended for sterilization, by reduction of the ambient pressure of the enclosed package 6. At a predetermined reduced ambient pressure, the communication with the vacuum source 16 is discontinued. The pipe 11 is placed in communication with the source 13 for sterilization gas through the connection 17 and the evacuated sterilization chamber 7' is filled with sterilization gas which, for purposes of sterilization, is brought into contact with the package 6 under the reduced ambient pressure. Thereafter, the connection 17 is closed and the pipe 11 is placed in communication with the tank 18 through the connection 14 for evacuation of spent sterilization gas from the sterilization chamber 7'. After this evacuation, the communication with the evacuation tank 18 is discontinued and the pipe 11 is finally placed in communication with the source 19 for sterile air through the connection 15. After pressure equalization in the sterilization chamber, the hood 7 is raised, as shown in Fig. 3, and the conveyor belt 5 is started and transports the sterilized package 6 to a subsequent station (not shown) for filling with sterilized food and sealing under aseptic conditions, at the same time as a following package is conveyed into the chamber 2 and the sterilization cycle is repeated.

Since the sterilization of the objects 6, in the described example the packages is carried out at reduced ambient pressure, an efficient sterilization will be achieved even at low gas flow rates, which implies that the sterilization in the method according to the invention may be carried out under considerably gentler sterilization conditions for the objects 6 than is the case in the prior art gas phase sterilization under which the sterilization gas must flow at a high rate in contact with the objects 6 in order to ensure an efficient contact from the point of view of sterilization between the gas and the objects 6.

## Claims

1. A method for sterilizing objects (6) by means of a gaseous sterilization agent wherein the boundary layer of ambient air adjacent the surfaces of the objects intended for sterilization is bringing into contact the gaseous sterilization agent with the objects using a vacuum source, characterized in
that the objects (6) are disposed onto a conveyor belt (5), that said conveyor belt (5) is moved into a treatment zone (2'), that a hood (7) is vertically displaced above said conveyor belt (5) and is lowered into a lowered position to sealingly abut against the conveyor belt (5) which serves as a chamber bottom, for the formation of an evacuable sterilization chamber (7') completely enclosing said object (6) and being connectable with a source (17) for the geaseous sterilization agent through an outer connection (13) after said chamber (7') is brought under reduced pressure by said vacuum source (16).

2. Method as claimed in claim 1,
**characterized in that**
the gaseous sterilization agent is caused to flow in contact with the object (6) at low flow rate.

3. Apparatus for sterilizing objects (6) by the method according to claim 1 or 2 comprising a vacuum source (16) connectable to the space in the vicinity of a belt (5) movable through a sterilization chamber (7') in which said sterilization agent can sterilize said objects (6),
**characterized in that**
a hood (7) is vertically displaceable above a conveyor belt (5) for the objects (6), the hood (7) being, in the lowered position, disposed to sealingly abut against the conveyor belt (5) serving as chamber bottom, for the formation of said sterilization chamber (7') completely enclosing said objects (6) and being connectable with a source (17) for said gaseous sterilization agent through an outer connection (13).

4. Apparatus as claimed in claim 3,
**characterized in that**
the outer connection (13) for the gaseous sterilization agent is connectable to the sterilization chamber (7') through one or more vertical inlet pipes (11) centrally disposed in the chamber (7').

5. Apparatus as claimed in claim 3 or 4,
**characterized in that**
the hood (7) is disposed within an outer, substantially wholly closed chamber casing (2) with inlet aperture (3) and outlet aperture (4) for the packages (6) advanced on the conveyor belt (5).

6. Apparatus as claimed in claim 4 or 5,
**characterized in that**
said inlet pipe (11) is connectable to a recycling tank (18) for said gaseous sterilization agent through an outer connection (14).

7. Apparatus as claimed in anyone of claims 4-6,
**characterized in that**
said inlet pipe (11) is connectable to a source (19) for sterile air through an outer connection (15).

## Patentansprüche

1. Verfahren zur Sterilisation von Gegenständen (6) mittels eines gasförmigen Sterilisationsmittels, wobei die Grenzschicht von Umgebungsluft angrenzend an die Flächen der zur Sterilisation vorgesehenen Gegenstände das gasförmige Sterilisationsmittel unter Verwendung einer Vakuumquelle mit den Gegenständen in Kontakt bringt,
dadurch gekennzeichnet,
daß die Gegenstände (6) auf einem Förderband (5) angeordnet werden, daß das Förderband (5) in eine Behandlungszone (2') bewegt wird, daß eine Haube (7) vertikal über dem Förderband (5) verschoben und in eine abgesenkte Position gesenkt wird, um dichtend gegen das als Kammer dienende Förderband (5) zur Bildung einer evakuierbaren Sterilisationskammer (7') in Anlage zu gelangen, die den Gegenstand (6) vollständig umgibt und über einen äußeren Anschluß (13) an eine Quelle (17) für das gasförmige Sterilisationsmittel angeschlossen werden kann, nachdem die Kammer (7') durch die Vakuumquelle (16) unter reduzierten Druck gebracht ist.

2. Verfahren nach Anspruch 1,
**dadurch gekennzeichnet**, **daß**
das gasförmige Sterilisationsmittel veranlaßt wird, mit einer niedrigen Strömungsrate in Kontakt mit dem Gegenstand (6) zu fließen.

3. Vorrichtung zur Sterilisation von Gegenständen (6) durch das Verfahren nach Anspruch 1 oder 2 mit einer Vakuumquelle (16), die an den Raum in der Nachbarschaft eines durch eine Sterilisationskammer (7') bewegbaren Bandes (5) anschließbar ist, in der das Sterilistationsmittel die Gegenstände (6) sterilisieren kann,
**dadurch gekennzeichnet**, **daß**
eine Haube (7) vertikal über einem Förderband (5) für die Gegenstände (6) verschiebbar ist, wobei die Haube (7) in der abgesenkten Position so angeordnet ist, daß sie dichtend gegen das als Kammerboden dienende Förderband (5) in Anlage gelangt, um die Sterilisationskammer (7') zu bilden, die die Gegenstände (6) vollständig umgibt, und über einen äußeren Anschluß (13) an eine Quelle (17) für das gasförmige Sterilisationsmittel anschließbar ist.

4. Vorrichtung nach Anspruch 3,
**dadurch gekennzeichnet**, **daß**
der äußere Anschluß (13) für das gasförmige Sterilisationsmittel über ein oder mehrere vertikale, zentral in der Kammer (7') angeordnete Einlaßrohre (11) an die Sterilisationskammer (7') anschließbar ist.

5. Vorrichtung nach Anspruch 3 oder 4,
**dadurch gekennzeichnet**, **daß**
die Haube (7) innerhalb eines äußeren, im wesentlichen vollständig geschlossenen Kammergehäuses (2) mit einer Einlaßöffnung (3) und einer Auslaßöffnung (4) für die auf dem Förderband (5) vorgeschobenen Packungen (6) angeordnet ist.

6. Vorrichtung nach Anspruch 4 oder 5,
**dadurch gekennzeichnet**, **daß**
das Einlaßrohr (11) über einen äußeren Anschluß (14) an einen Wiederverwertungstank (18) für das gasförmige Sterilisationsmittel anschließbar ist.

7. Vorrichtung nach einem der Ansprüche 4 - 6,
**dadurch gekennzeichnet**, **daß**
das Einlaßrohr (11) über einen äußeren Anschluß (15) an eine Quelle (19) für sterile Luft anschließbar ist.

## Revendications

1. Procédé pour stériliser des objets (6) au moyen d'un agent stérilisant gazeux, dans lequel la couche limite d' air ambiant proche des surfaces des objets à stériliser est mise en contact avec l'agent stérilisation gazeux en utilisant une source de vide, caractérisé en ce que les objets (6) sont disposés sur une courroie transporteuse (5), que ladite courroie transporteuse (5) se déplace jusque dans une zone de traitement (2'), qu'une hotte (7) est déplacée verticalement au-dessus de la courroie transporteuse (5) et est abaissée en position inférieure, de manière à buter de manière étanche contre la courroie transporteuse (5), qui sert alors de fond de la chambre, afin de former une chambre de stérilisation (7') entourant complètement ledit objet (6), cette chambre pouvant être mise sous vide et être connectée à une source (17) d'agent stérilisant gazeux par une connexion extérieure (13) après que ladite chambre (7') a été mise sous pression réduite par ladite source de vide (16).

2. Procédé selon la revendication 1, caractérisé en ce que l'agent stérilisant gazeux mis en contact avec l'objet (6) passe avec un faible débit sur celui-ci.

3. Appareil pour stériliser des objets (6) par le procédé selon la revendication 1 ou 2, comprenant une source de vide (16) pouvant être connectée à l'espace proche d'une courroie (5) pouvant pénétrer dans une chambre de stérilisation (7'), dans lequel ledit agent stérilisant peut stériliser lesdits objets (6), caractérisé en ce qu'une hotte (7) peut se déplacer verticalement au-dessus d'une courroie transporteuse (5) pour les objets (6), la hotte (7) étant, en position inférieure, disposée de manière à buter hermétiquement contre la courroie transporteuse (5) servant alors de fond pour la chambre, pour former ladite chambre de stérilisation (7') entourant complètement lesdits objets (6) et pouvant être connectée à une source (17) d'agent stérilisant gazeux par une connexion extérieure (13).

4. Appareil selon la revendication 3, caractérisé en ce que la connexion extérieure (13) pour l'agent stérilisant gazeux peut être connectée à la chambre de stérilisation (7') par un ou plusieurs tuyaux d'entrée verticaux (11) disposés au centre de la chambre (7').

5. Appareil selon la revendication 3 ou 4, caractérisé en ce que la hotte (7) est disposée dans un boîtier de chambre extérieur (2) essentiellement complètement fermé, avec une ouverture d'entrée (3), et une ouverture de sortie (4) pour les emballages (6) transportés sur la courroie transporteuse (5).

6. Appareil selon la revendication 4 ou 5, caractérisé en ce que ledit tuyau d'entrée (11) peut être connecté à un réservoir de recyclage (18) pour ledit agent stérilisant gazeux par une connexion extérieure (14).

7. Appareil selon l'une quelconque des revendications 4 à 6, caractérisé en ce que ledit tuyau d'entrée (11) peut être connecté à une source (19) d'air stérile par une connexion extérieure (15).
